(19) Europäisches Patentamt
European Patent Office
Office européen des brevets

(11) Veröffentlichungsnummer: **0 151 554**
**A1**

(12) # EUROPÄISCHE PATENTANMELDUNG

(21) Anmeldenummer: 85810028.2

(22) Anmeldetag: 28.01.85

(51) Int. Cl.⁴: C 07 D 239/46
C 07 D 239/52, C 07 D 251/16
C 07 D 251/46, A 01 N 47/36
//C07C161/00, C07C161/04

(30) Priorität: 03.02.84 CH 511/84

(43) Veröffentlichungstag der Anmeldung:
14.08.85 Patentblatt 85/33

(84) Benannte Vertragsstaaten:
BE CH DE FR GB IT LI NL

(71) Anmelder: CIBA-GEIGY AG
Postfach
CH-4002 Basel(CH)

(72) Erfinder: Töpfl, Werner, Dr.
Dorneckstrasse 68
CH-4143 Dornach(CH)

(72) Erfinder: Schurter, Rolf, Dr.
Holzmattstrasse 45
CH-4102 Binningen(CH)

(72) Erfinder: Meyer, Willy
Talweg 49
CH-4125 Riehen(CH)

(72) Erfinder: Böhner, Beat, Dr.
Hügelweg 3
CH-4102 Binningen(CH)

(54) **Neue Phenylsulfonamidderivate.**

(57) Phenylsulfonamidderivate der allgemeinen Formel

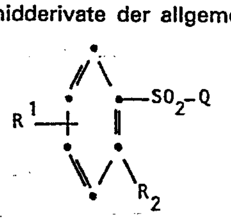

und die Salze dieser Verbindungen mit Aminen, Alkali- oder Erdalkalimetallbasen oder mit quaternären Ammoniumbasen haben gute pre- und postemergent-selektive herbizide und wuchsregulierende Eigenschaften.

In dieser Formel bedeuten

$R^1$ Wasserstoff, Fluor, Chlor, Brom, Nitro, $C_1$-$C_4$–Alkyl, $C_1$-$C_4$–Alkylthio, $C_1$-$C_4$–Alkoxy, Trifluormethyl, Trifluormethoxy, Difluormethoxy oder $C_1$-$C_4$–Alkoxycarbonyl,

$R^2$ eine Gruppe 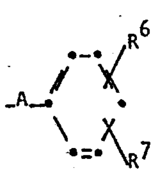

Q eine Gruppe 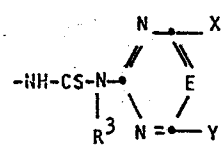 oder

eine Gruppe 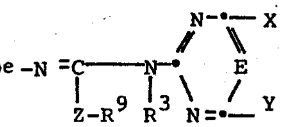

E Stickstoff oder die Methingruppe,

Z Sauerstoff oder Schwefel,

A Sauerstoff, Schwefel, eine –SO– oder –$SO_2$–Brücke oder eine Gruppe

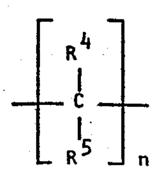

n die Zahl eins oder null,

$R^3$ Wasserstoff oder $C_1$-$C_4$–Alkyl,

$R^4$ und $R^5$ unabhängig voneinander Wasserstoff oder $C_1$-$C_4$–Alkyl,

./...

EP 0 151 554 A1

$R^6$ Wasserstoff, $C_1$-$C_4$-Alkyl, $C_1$-$C_4$-Alkoxy, Halogen, Trifluormethyl, Nitro, $COOR^8$ oder durch Halogen substituiertes $C_1$-$C_4$-Alkoxy,

$R^7$ Wasserstoff, Fluor, Chlor, Brom, $C_1$-$C_4$-Alkyl oder $C_1$-$C_4$-Alkoxy,

$R^9$ $C_1$-$C_6$-Alkyl, $C_3$-$C_6$-Alkenyl, $C_3$-$C_6$-Alkinyl, $C_2$-$C_6$-Halogenalkyl, $C_2$-$C_6$-Alkoxyalkyl, Benzyl, Phenyl, eine Gruppe $-CHR^{10}-COOR^{11}$, $-CHR^{10}-CN$, $-CHR^{10}-CONR^{12}R^{13}$, $-(CH_2)_m-NR^{12}R^{13}$ oder durch $C_1$-$C_4$-Alkyl, Fluor, Chlor, Brom, Nitro, Trifluormethyl, Trifluormethoxy oder $C_1$-$C_4$-Alkoxy substituiertes Benzyl oder Phenyl,

m eine Zahl von zwei bis sechs,

$R^{10}$ Wasserstoff oder $C_1$-$C_4$-Alkyl,

$R^8$ und $R^{11}$ unabhängig voneinander Wasserstoff, $C_1$-$C_4$-Alkyl, $C_2$-$C_4$-Alkenyl, $C_3$-$C_4$-Alkinyl oder durch $C_1$-$C_4$-Alkoxy, Fluor, Chlor, Brom oder Phenyl $C_1$-$C_4$-Alkyl,

$R^{12}$ Wasserstoff, $C_1$-$C_4$-Alkyl, $C_2$-$C_4$-Alkenyl oder $C_3$-$C_4$-Alkinyl,

$R^{13}$ Wasserstoff, $C_1$-$C_4$-Alkyl, $C_2$-$C_4$-Alkenyl, $C_3$-$C_4$-Alkinyl, $C_1$-$C_4$-Alkoxy, $C_3$-$C_6$-Cycloalkyl, Phenyl oder Benzyl,

$R^{14}$ und $R^{15}$ unabhängig voneinander Wasserstoff oder $C_1$-$C_4$-Alkyl und

X und Y unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder $-NR^{14}R^{15}$ bedeuten, mit der Massgabe, dass keiner der Reste X und Y für Difluormethoxy steht, wenn die Gruppe Q für

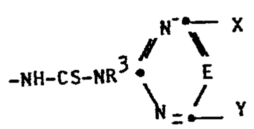

steht.

0151554

5-14760/+

CIBA-GEIGY AG
Basel (Schweiz)

# Neue Phenylsulfonamidderivate

Die vorliegende Erfindung betrifft neue, herbizid wirksame und pflanzenwuchsregulierende Phenylsulfonamidderivate, Verfahren zu ihrer Herstellung, die sie als Wirkstoffe enthaltenden Mittel sowie deren Verwendung zum Bekämpfen von Unkräutern, vor allem selektiv in Nutzpflanzenkulturen oder zum Regulieren und Hemmen des Pflanzenwachstums.

Die erfindungsgemässen Phenylsulfonamidderivate entsprechen der Formel I

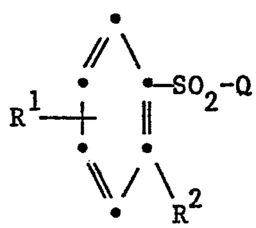

(I)

worin

$R^1$ Wasserstoff, Fluor, Chlor, Brom, Nitro, $C_1$-$C_4$-Alkyl, $C_1$-$C_4$-Alkylthio, $C_1$-$C_4$-Alkoxy, Trifluormethyl, Trifluormethoxy, Difluormethoxy oder $C_1$-$C_4$-Alkoxycarbonyl,

$R^2$ eine Gruppe 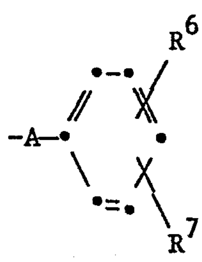,

Q eine Gruppe 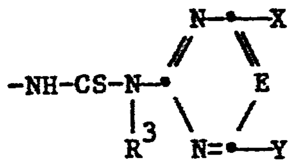 oder

eine Gruppe 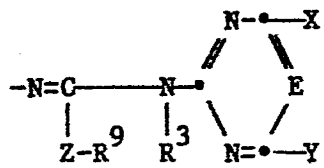 ,

E Stickstoff oder die Methingruppe,

Z Sauerstoff oder Schwefel

A Sauerstoff, Schwefel, eine -SO- oder $-SO_2-$Brücke oder eine Gruppe

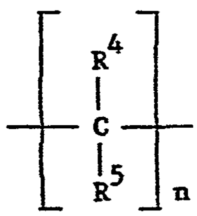 ,

n die Zahl eins oder null,

$R^3$ Wasserstoff oder $C_1$-$C_4$-Alkyl,

$R^4$ und $R^5$ unabhängig voneinander Wasserstoff oder $C_1$-$C_4$-Alkyl,

$R^6$ Wasserstoff, $C_1$-$C_4$-Alkyl, $C_1$-$C_4$-Alkoxy, Halogen, Trifluormethyl, Nitro, $COOR^8$ oder durch Halogen substituiertes $C_1$-$C_4$-Alkoxy,

$R^7$ Wasserstoff, Fluor, Chlor, Brom, $C_1$-$C_4$-Alkyl oder $C_1$-$C_4$-Alkoxy,

$R^9$ $C_1$-$C_6$-Alkyl, $C_3$-$C_6$-Alkenyl, $C_3$-$C_6$-Alkinyl, $C_2$-$C_6$-Halogenalkyl, $C_2$-$C_6$-Alkoxyalkyl, Benzyl, Phenyl, eine Gruppe $-CHR^{10}-COOR^{11}$, $-CHR^{10}-CN$, $-CHR^{10}-CONR^{12}R^{13}$, $-(CH_2)_m-NR^{12}R^{13}$ oder durch $C_1$-$C_4$-Alkyl, Fluor, Chlor, Brom, Nitro, Trifluormethyl, Trifluormethoxy oder $C_1$-$C_4$-Alkoxy substituiertes Benzyl oder Phenyl,

m eine Zahl von zwei bis sechs,

$R^{10}$ Wasserstoff oder $C_1$-$C_4$-Alkyl,

$R^8$ und $R^{11}$ unabhängig voneinander Wasserstoff, $C_1$-$C_4$-Alkyl, $C_2$-$C_4$-Alkenyl, $C_3$-$C_4$-Alkinyl oder durch $C_1$-$C_4$-Alkoxy, Fluor, Chlor, Brom oder Phenyl substituiertes $C_1$-$C_4$-Alkyl,

$R^{12}$ Wasserstoff, $C_1$-$C_4$-Alkyl, $C_2$-$C_4$-Alkenyl oder $C_3$-$C_4$-Alkinyl,

$R^{13}$ Wasserstoff, $C_1$-$C_4$-Alkyl, $C_2$-$C_4$-Alkenyl, $C_3$-$C_4$-Alkinyl, $C_1$-$C_4$-Alkoxy, $C_3$-$C_6$-Cycloalkyl, Phenyl oder Benzyl,

$R^{14}$ und $R^{15}$ unabhängig voneinander Wasserstoff oder $C_1$-$C_4$-Alkyl und

X und Y unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder $-NR^{14}R^{15}$ bedeuten,

mit der Massgabe, dass keiner der Reste X und Y für Difluormethoxy steht, wenn die Gruppe Q für

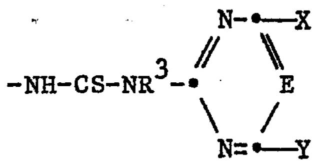

steht,

sowie den Salzen dieser Verbindungen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentanmeldungen 44211, 44807, 44808, 56969 und 74595 sowie der japanischen Patentpublikation 58 126 872 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl. Unter Alkoxy ist zu verstehen: Methoxy, Aethoxy, n-Propyloxy, i-Propyloxy oder die vier isomeren Butyloxy, insbesondere aber Methoxy, Aethoxy oder i-Propyloxy. Beispiele für Alkylthio sind Methylthio, Aethylthio, n-Propylthio, i-Propylthio oder die vier isomeren Butylthio, insbesondere aber Methylthio und Aethylthio.

Unter Halogen in den Definitionen als Teil eines Substituenten wie in Halogenalkoxy oder Halogenalkyl sind Fluor, Chlor und Brom, vorzugsweise jedoch Fluor und Chlor zu verstehen. Halogenalkyl selbst oder als Teil von Halogenalkoxy steht in der Regel für

Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3-3,3-Hexafluorpropyl, insbesondere aber Fluormethyl, Chlormethyl, Difluormethyl und Trifluormethyl. Alkenyl steht zum Beispiel für Vinyl, Allyl, Methallyl, 3- oder 2-Butenyl. Alkinyl bedeutet vorzugsweise Propargyl, 2- oder 3-Butinyl. Unter Cycloalkyl ist im allgemeinen Cyclopropyl, Cyclobutyl, Cyclopentyl oder Cyclohexyl zu verstehen. Unter den substituierten Phenyl- und Benzylresten, die für $R^9$ stehen können, sind Halogenphenyl und Halogenbenzyl bevorzugt, hierunter wiederum Fluor- oder Chlorphenyl oder -benzyl.

Die unter $R^2$ definierte Gruppe bildet beispielsweise folgende Grundkörper: Phenyl, Phenoxy, Phenylthio, Phenylsulfinyl, Phenylsulfonyl oder Benzyl. Bevorzugt sind: Phenyl, Phenoxy und Benzyl.

Durch die Variation der Gruppe Q verkörpert die Formel I die folgenden Grundstrukturen von Verbindungen: N-Phenylsulfonyl-N'-pyrimidinyl- oder -triazinyl-thioharnstoffe, N-Phenylsulfoynl-N'-pyrimidinyl- oder -triazinyl-isoharnstoffe und N-Phenylsulfonyl-N'-pyrimidinyl- oder -triazinyl-isothioharnstoff.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin,

Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) $R^1$ Wasserstoff oder Chlor bedeutet oder
b) $R^3$ Wasserstoff oder Methyl bedeutet oder
c) $R^6$ Wasserstoff oder Halogen und $R^7$ Wasserstoff bedeuten oder
d) X und Y unabhängig voneinander $C_1$-$C_4$-Alkyl, $C_1$-$C_4$-Alkoxy oder $C_1$-$C_4$-Halogenalkoxy bedeuten und zusammen höchstens 5 Kohlenstoffatome enthalten oder
e) A die direkte Bindung, Sauerstoff oder eine Methylenbrücke bedeutet.

Eine besonders bevorzugte Untergruppe von Verbindungen der Formel I bilden diejenigen Wirkstoffe, in denen $R^1$ Wasserstoff oder Chlor, $R^3$ Wasserstoff oder Methyl, $R^6$ Wasserstoff oder Halogen, $R^7$ Wasserstoff, A die direkte Bindung, Sauerstoff oder eine Methylenbrücke und X und Y unabhängig voneinander $C_1$-$C_4$-Alkyl, $C_1$-$C_4$-Alkoxy oder $C_1$-$C_4$-Halogenalkoxy bedeuten, wobei X und Y zusammen höchstens 5 Kohlenstoffatome enthalten.

Als bevorzugte Einzelverbindungen sind zu nennen:
N-(2-Phenyl-phenylsulfonyl)-N'-(4,6-dimethoxypyrimidin-2-yl)-S-methyl-isothioharnstoff.
N-(2-Phenyl-phenylsulfonyl)-N'-(4,6-dimethoxypyrimidin-2-yl)-thioharnstoff,

N-(2-Phenyl-phenylsulfonyl)-N'-(4-methoxy-6-methyl-pyrimidin-2-yl)-thioharnstoff,
N-(2-Phenyl-phenylsulfonyl)-N'-(4,6-dimethoxy-triazin-2-yl)-thioharnstoff und
N-(2-Phenyl-phenylsulfonyl)-N'-(4-methoxy-6-methyl-triazin-2-yl)-thioharnstoff.

Die Herstellung der Verbindungen der Formel I erfolgt im allgemeinen nach den folgenden Methoden.

Nach einem ersten Verfahren werden die Verbindungen der Unterformel Ia

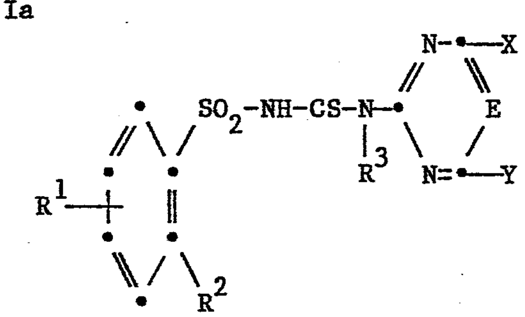

(Ia),

worin $R^1$, $R^2$, $R^3$, E, X und Y die unter Formel I gegebenen Bedeutungen haben, erhalten, indem man ein substituiertes Phenylsulfonamid der Formel II

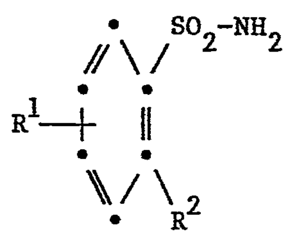

(II),

worin $R^1$ und $R^2$ die unter Formel I gegebenen Bedeutungen haben, in Gegenwart einer Base mit einem Dithiocarbamat der Formel III

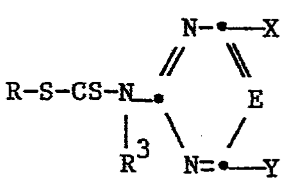

(III),

worin $R^3$, E, X und Y die unter Formel I gegebenen Bedeutungen haben und R für Phenyl, Alkyl oder substituiertes Phenyl steht, umsetzt.

Nach einem zweiten Verfahren gelangt man zu Verbindungen der Formel Ia, indem man ein Phenylsulfonyldithiocarbamat der Formel IV

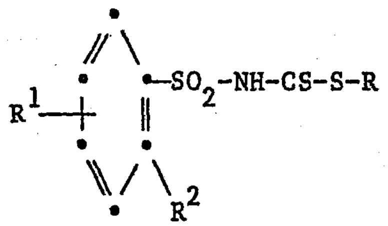

(IV),

worin $R^1$ und $R^2$ die unter Formel I gegebenen Bedeutungen haben und R für Phenyl, Alkyl oder substituiertes Phenyl steht, mit einem Amin der Formel V

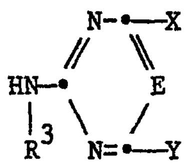

(V),

worin $R^3$, E, X und Y die unter Formel I gegebenen Bedeutungen haben, umsetzt.

Schliesslich kann man die Verbindungen der Formel Ia auch erhalten, indem man ein Sulfonylisothiocyanat der Formel VI

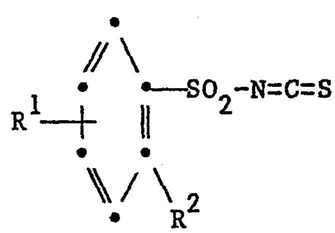

(VI),

worin $R^1$ und $R^2$ die unter Formel I gegebenen Bedeutungen haben, mit einem Amin der oben angegebenen Formel V umsetzt.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie

Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo-[2.2.2]octan, 1,5-Diazabicyclo[4.3.0]non-5-en oder 1,5-Diazabicyclo[5.4.0]undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Verbindungen der Unterformel Ib

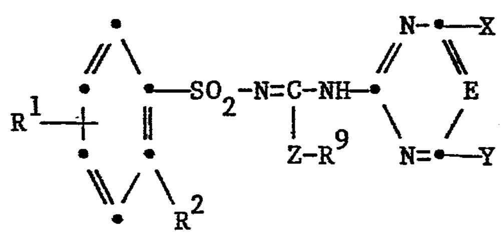

(Ib) ,

worin $R^1$, $R^2$, $R^9$, E, X, Y und Z die unter Formel I gegebenen Bedeutungen haben, können hergestellt werden, indem man einen Sulfonylharnstoff der Formel VII

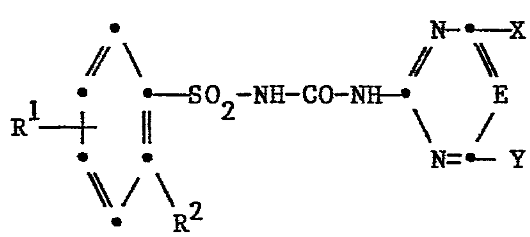

(VII) ,

worin $R^1$, $R^2$, E, X und Y die unter Formel I gegebenen Bedeutungen haben, durch Umsetzung mit einem Dehydratisierungsreagenz in ein Carbodiimid der Formel VIII

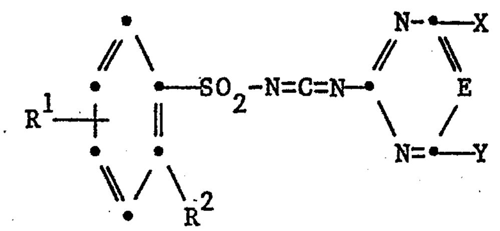

(VIII),

worin $R^1$, $R^2$, E, X und Y die unter Formel I gegebenen Bedeutungen haben, überführt und dieses mit einem Alkohol oder Mercaptan der Formel IX

$$H - Z - R^9 \quad (IX),$$

worin $R^9$ und Z die unter Formel I gegebenen Bedeutungen haben, umsetzt.

Die Verbindungen der Formel Ib können gewünschtenfalls durch Umsatz mit einem Alkylierungsmittel in Gegenwart einer Base am Brückenstickstoffatom alkyliert werden (Einführung des Substituenten $R^3$). Uebliche Alkylierungsmittel sind im Handel erhältlich, wie Alkylhalogenide, wie Methyljodid, Methylbromid oder Methylchlorid oder wie Ester starker Säuren wie Dimethylsulfat.

Als geeignete Dehydratisierungsreagenzien (VII → VIII) haben sich beispielsweise Phospohorverbindungen erwiesen wie: Triphenylbromphosphoniumbromid, Trimethylbromphosphoniumbromid, Triäthylbromphosphoniumbromid, Tributylbromphosphoniumbromid oder Phenoxyphosphorsäuredichlorid, Phenoxyphosphorsäuredibromid, Diphenoxyphosphorsäurechlorid oder Phenoxychlorphosphorsäure-phenylamid. Die entstandenen Carbodiimide der Formel VIII können isoliert werden. Vorteilhaft ist es jedoch, die Reaktionslösung, die das Carbodiimid enthält, direkt ohne Isolierung mit dem Alkohol oder Mercaptan der Formel IX umzusetzen. Im allgemeinen ist es von Vorteil, wenn man die beiden Reaktionsschritte (VII → VIII und VIII → Ib) in inerten

Lösungsmitteln ausführt. Isoliert man das Carbodiimid-Zwischenprodukt nicht, so ist es zweckmässig, beide Stufen im gleichen Lösungsmittel durchzuführen. Geeignete Lösungsmittel sind beispielsweise Tetrachlorkohlenstoff, Chloroform, Tetrachloräthan, Trichloräthan, Methylenchlorid, Dioxan, Diäthyläther, Tetrahydrofuran, Chlorbenzol, Toluol oder Xylol oder Gemische davon. Die Reaktionstemperatur liegt bei beiden Reaktionsschritten im allgemeinen zwischen -15°C und +60°C, insbesondere zwischen 0°C und 30°C. Als geeignete Basen für die Dehydratisierungsreaktion (VII -> VIII) haben sich tertiäre Amine wie Trimethylamin, Triäthylamin, Chinuclidin, 1,4-Diazabicyclo-[2.2.2]octan, Diazabicyclo[4.3.0]non-5-en, 1,5-Diazabicyclo[5.4.0]-undec-7-en, Chinolin oder Pyridin erwiesen.

Weiter erhält man Verbindungen der Unterformel Ic

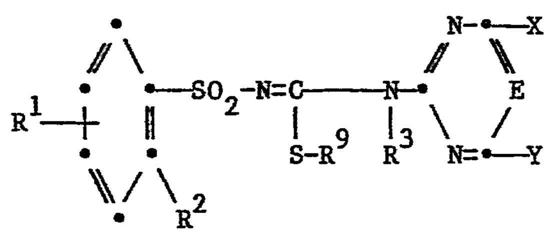

(Ic),

worin $R^1$, $R^2$, $R^3$, $R^9$, E, X und Y die unter Formel I gegebenen Bedeutungen haben, indem man ein Sulfonamid der Formel II

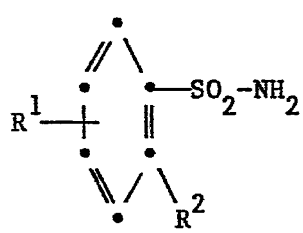

(II),

worin $R^1$ und $R^2$ die unter Formel I gegebenen Bedeutungen haben, in Gegenwart einer Base zunächst durch Umsetzung mit Schwefelkohlenstoff und anschliessend einem Mercaptan der Formel IXa

$$H - S - R^9 \quad \text{(IXa)}$$

in eine Verbindung der Formel X

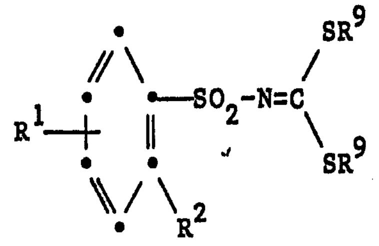

(X),

worin $R^1$, $R^2$ und $R^9$ die unter Formel I gegebenen Bedeutungen haben, überführt und dieses Zwischenprodukt mit einem Aminsalz der Formel XI

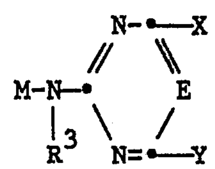

(XI),

worin $R^3$, E, X und Y die unter Formel I gegebenen Bedeutungen haben und M für ein Metallatom oder ein Aequivalent eines Metallatoms, vorzugsweise Lithium, Natrium, Kalium, Calcium oder Magnesium, steht, umsetzt.

Schliesslich sind die Verbindungen der Unterformel Id

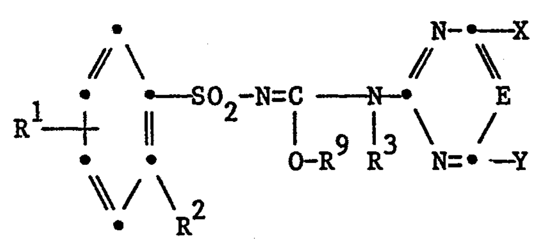

(Id),

worin $R^1$, $R^2$, $R^3$, $R^9$, E, X und Y die unter Formel I gegebenen Bedeutungen haben, nach der folgenden im Schema 1 skizzierten Reaktionsfolge erhältlich:

Schema 1:

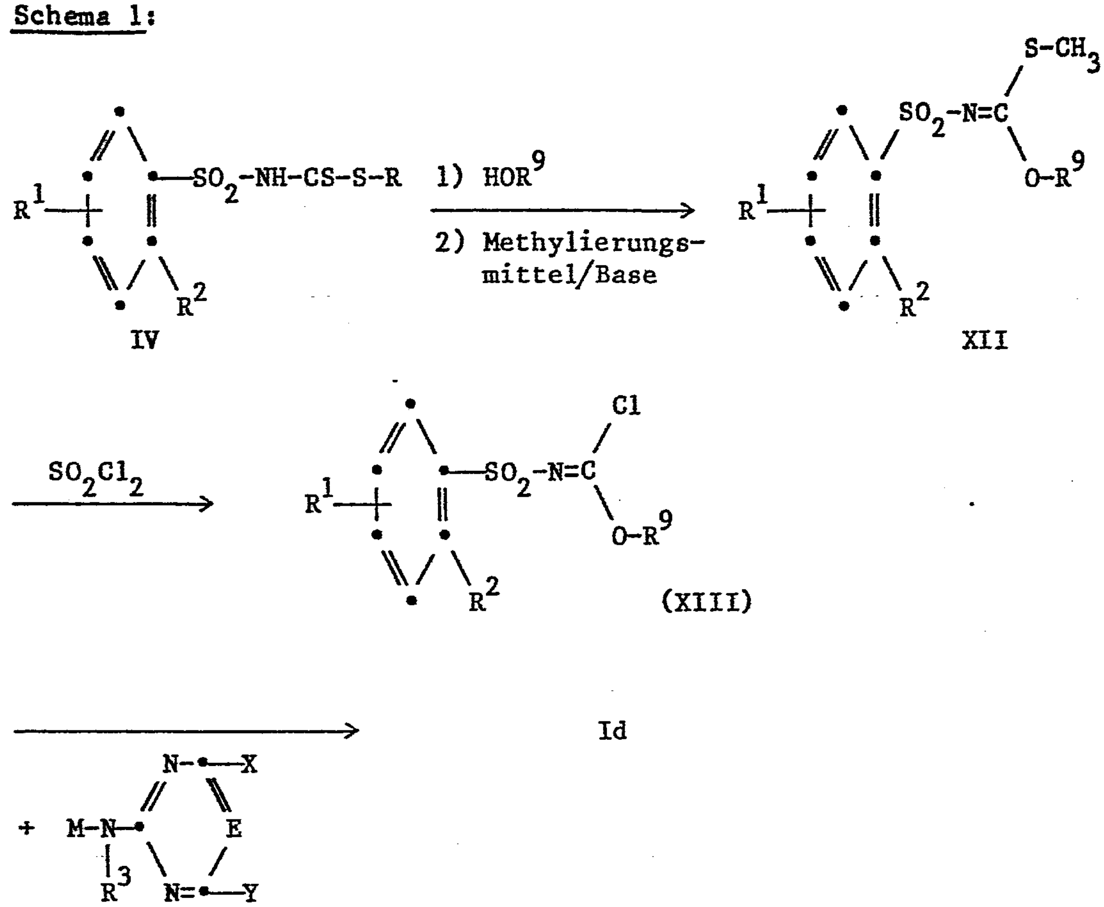


IV

XII

(XIII)

Id

XI

Die Zwischenprodukte der Formel VIII sind neu. Sie wurden speziell für die Synthese von Verbindungen der Formel I entwickelt. Diese Verbindungen bilden einen weiteren Bestandteil der vorliegenden Erfindung.

Die Zwischenprodukte der Formel VI sind neu und können nach dem im folgenden Schema 2 dargestellten Verfahren hergestellt werden:

Schema 2:

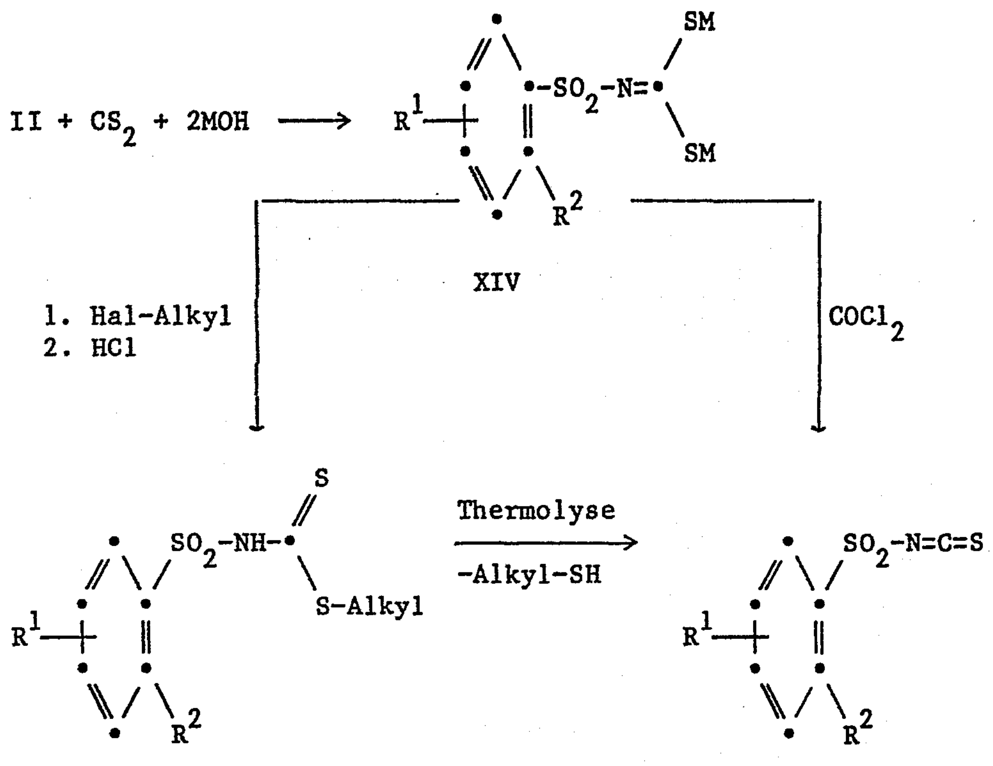


IV VI

Dabei wird in einem ersten Schritt das Sulfonamid II mit Schwefelkohlenstoff und in Gegenwart einer Base MOH in das N-Sulfonyl-iminodithiocarbonat der Formel XIV umgewandelt, und dann, in einem zweiten Schritt, entweder über das N-Sulfonyldithiocarbamat IV und anschliessende Thermolyse, oder direkt durch Umsetzung mit Phosgen das Sulfonylisothiocyanat VI hergestellt.

Die Ausgangsmaterialien der Formeln II, III, IV, V, VII, IX, X, XI, XII und XIII sind aus der Literatur bekannt oder können analog zu bekannten Verfahren hergestellt werden.

Die erhaltenen Phenylsulfonamidderivate der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Wirkstoffe der Formel I sind stabile Verbindungen. Ihre Handhabung bedarf keiner vorsorglichen Massnahme.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Zuckerrüben und ganz besonders Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von

natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 % |

Benetzbares Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele:

Beispiel H1: N-(2-Phenyl-phenylsulfonyl)-N'-(4,6-dimethoxypyrimidin-2-yl)-S-methyl-isothioharnstoff (Verb. Nr. 2.03).

a) N-(2-Phenyl-phenylsulfonyl)-imino-dithiokohlensäure-S,S-dimethylester.

Zu einer Lösung von 10.7 g 2-Phenyl-phenylsulfonamid in 50 ml Dimethylformamid lässt man gleichzeitig unter Kühlung 3,5 g Schwefelkohlenstoff und 7,4 g 50%ige Natronlauge zutropfen. Nachdem die entstandene gelbrote Lösung für 4 Stunden bei einer Temperatur zwischen 20°C und 25°C gehalten worden ist, setzt man unter Kühlung tropfenweise 11,6 g Dimethylsulfat zu. Nach 14 Stunden bei 20°C bis 25°C wird die gelbliche Lösung mit 1 l Wasser verdünnt. Das abgeschiedene Oel wird aus Methanol umkristallisiert. Man erhält so 13,0 g (84 % d.Th.) N-(2-Phenyl-phenylsulfonyl)-imino-dithiokohlensäure-S,S-dimethylester, Smp. 112-114°C.

b) Eine Lösung von 5,5 g 2-Amino-4,6-dimethoxypyrimidin in 150 ml Tetrahydrofuran wird mit 8,2 g Kalium-tert.butylat versetzt. Die gelbliche Suspension wird für 2 Stunden bei einer Temperatur zwischen 20°C und 25°C gerührt. Anschliessend trägt man in diese Suspension 12,0 g N-(2-Phenylphenylsulfonyl)-imino-dithiokohlensäure-S,S-dimethylester portionsweise ein und rührt für weitere 18 Stunden bei gleicher Temperatur. Nach Verdünnen mit 1 l Wasser wird die klare gelbe Lösung durch Zusatz von 9,0 g 36%iger Salzsäure angesäuert. Das abgeschiedene Oel wird aus Acetonitril umkristallisiert. Man erhält so 8,0 g (50 % d.Th.) N-(2-Phenylphenylsulfonyl)-N'-(4,6-dimethoxypyrimidin-2-yl)-S-methyl-isothioharnstoff, Smp. 133-135°C.

Beispiel H2: N-(2-Phenyl-phenylsulfonyl)-N'-(4,6-dimethoxy-pyrimidin-2-yl)-thioharnstoff (Verb. Nr. 1.03).

a) Dikalium-N-(2-phenyl-phenylsulfonyl)-imino-dithiocarbonat.
Zu einer Lösung von 70,0 g (0.30 ml) 2-Phenyl-phenylsulfonamid in 150 ml Dimethylformamid gibt man 18,15 g (0,30 mol) Schwefelkohlenstoff und anschliessend unter Kühlung bei 0°C 16,8 g (0,30 mol) pulverisiertes Kaliumhydroxid. Nach 1 1/2 Stunden werden nochmals 16,8 g (0,30 mol) Kaliumhydroxid zugefügt. Das Reaktionsgemisch wird dann 16 Stunden bei Raumtemperatur gerührt. Nachdem man 250 ml Aethylacetat hinzugefügt hat wird der erhaltene Niederschlag filtriert und mit Aethylacetet gewaschen und anschliessend getrocknet. Man erhält 91,0 g Dikalium-N-(2-phenyl-phenylsulfonyl)-imino-dithiocarbonat, Smp. 245°C (Zers.).

b) 2-Phenyl-phenylsulfonyl-isothiocyanat
91,0 g Dikalium-N-(2-phenyl-phenylsulfonyl)-iminodithiocarbonat werden in 500 ml Dioxan suspendiert. Dann werden unter Kühlung auf 0°C 30 g Phosgen eingeleitet. Darauf wird das Reaktionsgemisch 16 Std. bei Raumtemperatur gerührt. Anschliessend wird das entstandene Kaliumchlorid abfiltriert und das Filtrat eingeengt. Das erhaltene, ölige 2-Phenyl-phenylsulfonyl-isothiocyanat wird direkt weiterverwendet.

c) 3,1 g (0,020 mol) 2-Amino-4,6-dimethoxy-pyrimidin, 30 ml Acetonitril und 5,51 g (0,020 mol) 2-Phenyl-phenylsulfonyl-isothiocyanat werden 16 Stunden bei Raumtemperatur gerührt. Dann wird der Niederschlag filtriert, mit wenig Acetonitril gewaschen und getrocknet. Man erhält 5,9 g N-(2-Phenyl-phenylsulfonyl)-N'-(4,6-dimethoxy-pyrimidin-2-yl)-thioharnstoff, Smp. 200-201°C (Zers.).

In analoger Weise werden die in den angeschlossenen Tabellen aufgelisteten Zwischen- und Endprodukte hergestellt.

Tabelle 1

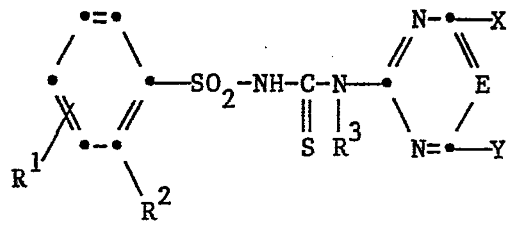

| Nr. | $R^2$ | $R^1$ | $R^3$ | X | Y | E | Physikal. Daten |
|---|---|---|---|---|---|---|---|
| 1.01 | Phenyl | H | H | $CH_3$ | $CH_3$ | CH | |
| 1.02 | Phenyl | H | H | $CH_3$ | $OCH_3$ | CH | Smp.187°C (Zers.) |
| 1.03 | Phenyl | H | H | $OCH_3$ | $OCH_3$ | CH | Smp.200°C (Zers.) |
| 1.04 | Phenyl | H | $CH_3$ | $OCH_3$ | $OCH_3$ | CH | |
| 1.05 | Phenyl | H | $CH_3$ | $CH_3$ | $OCH_3$ | CH | |
| 1.06 | Phenyl | 6-Cl | H | $CH_3$ | $OCH_3$ | CH | |
| 1.07 | Phenyl | 6-Cl | H | $OCH_3$ | $OCH_3$ | CH | |
| 1.08 | Phenyl | H | H | $CH_3$ | $CH_3$ | N | |
| 1.09 | Phenyl | H | H | $CH_3$ | $OCH_3$ | N | |
| 1.10 | Phenyl | H | H | $OCH_3$ | $OCH_3$ | N | |
| 1.11 | Phenyl | 6-Cl | H | $CH_3$ | $OCH_3$ | N | |
| 1.12 | Phenyl | 6-Cl | H | $OCH_3$ | $OCH_3$ | N | |
| 1.13 | Phenyl | H | $CH_3$ | $CH_3$ | $OCH_3$ | N | |
| 1.14 | Phenyl | H | $CH_3$ | $OCH_3$ | $OCH_3$ | N | |
| 1.15 | 2-Fluorphenyl | H | H | $CH_3$ | $CH_3$ | CH | |
| 1.16 | 2-Fluorphenyl | H | H | $CH_3$ | $OCH_3$ | CH | |
| 1.17 | 2-Fluorphenyl | H | H | $OCH_3$ | $OCH_3$ | CH | |
| 1.18 | 2-Fluorphenyl | H | H | $CH_3$ | $CH_3$ | N | |
| 1.19 | 2-Fluorphenyl | H | H | $CH_3$ | $OCH_3$ | N | |

Tabelle 1 (Fortsetzung)

| Nr. | $R^2$ | $R^1$ | $R^3$ | X | Y | E | Physikal. Daten |
|---|---|---|---|---|---|---|---|
| 1.20 | 2-Fluorphenyl | H | H | $OCH_3$ | $OCH_3$ | N | |
| 1.21 | 2-Fluorphenyl | 6-Cl | H | $OCH_3$ | $OCH_3$ | CH | |
| 1.22 | 2-Fluorphenyl | 6-Cl | H | $CH_3$ | $OCH_3$ | N | |
| 1.23 | 2-Fluorphenyl | 6-Cl | H | $OCH_3$ | $OCH_3$ | N | |
| 1.24 | Phenoxy | H | H | $CH_3$ | $CH_3$ | CH | |
| 1.25 | Phenoxy | H | H | $CH_3$ | $OCH_3$ | CH | |
| 1.26 | Phenoxy | H | H | $OCH_3$ | $OCH_3$ | CH | |
| 1.27 | Phenoxy | H | H | $CH_3$ | $CH_3$ | N | |
| 1.28 | Phenoxy | H | H | $CH_3$ | $OCH_3$ | N | |
| 1.29 | Phenoxy | H | H | $OCH_3$ | $OCH_3$ | N | |
| 1.30 | Phenoxy | 6-Cl | H | $CH_3$ | $OCH_3$ | CH | |
| 1.31 | Phenoxy | 6-Cl | H | $OCH_3$ | $OCH_3$ | CH | |
| 1.32 | Phenoxy | 6-Cl | H | $CH_3$ | $OCH_3$ | N | |
| 1.33 | Phenoxy | 6-Cl | H | $OCH_3$ | $OCH_3$ | N | |
| 1.34 | 2-Fluorphenoxy | H | H | $CH_3$ | $CH_3$ | CH | |
| 1.35 | 2-Fluorphenoxy | H | H | $CH_3$ | $OCH_3$ | CH | |
| 1.36 | 2-Fluorphenoxy | H | H | $OCH_3$ | $OCH_3$ | CH | |
| 1.37 | 2-Fluorphenoxy | H | H | $CH_3$ | $CH_3$ | N | |
| 1.38 | 2-Fluorphenoxy | H | H | $CH_3$ | $OCH_3$ | N | |
| 1.39 | 2-Fluorphenoxy | H | H | $OCH_3$ | $OCH_3$ | N | |
| 1.40 | Benzyl | H | H | $CH_3$ | $CH_3$ | CH | |
| 1.41 | Benzyl | H | H | $CH_3$ | $OCH_3$ | CH | |
| 1.42 | Benzyl | H | H | $OCH_3$ | $OCH_3$ | CH | |

Tabelle 1 (Fortsetzung)

| Nr. | $R^2$ | $R_1$ | $R_3$ | X | Y | E | Physikal. Daten |
|---|---|---|---|---|---|---|---|
| 1.43 | Benzyl | H | H | $CH_3$ | $OCH_3$ | N | |
| 1.44 | Benzyl | H | H | $CH_3$ | $OCH_3$ | N | |
| 1.45 | Benzyl | H | H | $OCH_3$ | $OCH_3$ | N | |
| 1.46 | Phenyl | H | H | $SCH_3$ | $SCH_3$ | CH | |
| 1.47 | Phenyl | H | H | $OCH_3$ | $CH_2OCH_3$ | CH | |
| 1.48 | Phenyl | H | H | Cyclo-propyl | $OCH_3$ | CH | |
| 1.49 | Phenyl | H | H | $OCH_3$ | $OCF_2Br$ | CH | |
| 1.50 | Phenyl | H | H | $OCH_3$ | $OCH_2CH_3$ | CH | |
| 1.51 | Phenyl | H | H | $CH_2F$ | $OCH_3$ | CH | |
| 1.52 | Phenyl | H | H | $OCH_3$ | $SCH_3$ | CH | |
| 1.53 | Phenyl | H | H | $OCH_3$ | $SCHF_2$ | CH | |
| 1.54 | Phenyl | H | H | $OCH_3$ | $OCH_2CF_3$ | N | |
| 1.55 | Phenyl | H | H | $OCH_3$ | $OCH_2CH_3$ | N | |
| 1.56 | Phenyl | H | H | $OCH_3$ | $SCH_3$ | N | |
| 1.57 | Phenyl | 6-$OCH_3$ | H | $OCH_3$ | $OCH_3$ | CH | |
| 1.58 | Phenyl | 6-$OCH_3$ | H | $CH_3$ | $OCH_3$ | CH | |
| 1.59 | Phenyl | 6-F | H | $OCH_3$ | $OCH_3$ | CH | |
| 1.60 | Phenyl | 6-$CH_3$ | H | $OCH_3$ | $OCH_3$ | CH | |
| 1.61 | Phenyl | 6-$CF_3$ | H | $OCH_3$ | $OCH_3$ | N | |
| 1.62 | Phenyl | 6-$OCHF_2$ | H | $OCH_3$ | $OCH_3$ | CH | |
| 1.63 | Phenyl | H | H | $OCH_3$ | $N(CH_3)_2$ | N | |

Tabelle 2

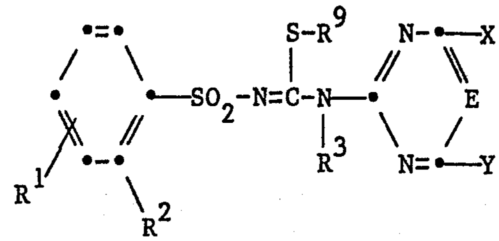

| Nr. | $R^2$ | $R^1$ | $R^3$ | $R^9$ | X | Y | E | Physikal. Daten |
|---|---|---|---|---|---|---|---|---|
| 2.01 | Phenyl | H | H | $CH_3$ | $CH_3$ | $CH_3$ | CH | |
| 2.02 | Phenyl | H | H | $CH_3$ | $CH_3$ | $OCH_3$ | CH | |
| 2.03 | Phenyl | H | H | $CH_3$ | $OCH_3$ | $OCH_3$ | CH | Smp. 133-135°C |
| 2.04 | Phenyl | H | $CH_3$ | $CH_3$ | $OCH_3$ | $OCH_3$ | CH | |
| 2.05 | Phenyl | 6-Cl | H | $CH_3$ | $OCH_3$ | $OCH_3$ | CH | |
| 2.06 | Phenyl | H | H | $CH_3$ | $CH_3$ | $CH_3$ | N | |
| 2.07 | Phenyl | H | H | $CH_3$ | $CH_3$ | $OCH_3$ | N | |
| 2.08 | Phenyl | H | H | $CH_3$ | $OCH_3$ | $OCH_3$ | N | |
| 2.09 | Phenyl | 6-Cl | H | $CH_3$ | $OCH_3$ | $OCH_3$ | N | |
| 2.10 | Phenyl | H | H | $C_2H_5$ | $CH_3$ | $OCH_3$ | CH | |

Tabelle 2 (Fortsetzung)

| Nr. | $R^2$ | $R^1$ | $R^3$ | $R^9$ | X | Y | E | Physikal. Daten |
|---|---|---|---|---|---|---|---|---|
| 2.11 | Phenyl | H | H | $C_2H_5$ | $OCH_3$ | $OCH_3$ | CH | Smp.148-150°C |
| 2.12 | Phenyl | H | H | $C_2H_5$ | $CH_3$ | $OCH_3$ | N | |
| 2.13 | Phenyl | H | H | $C_2H_5$ | $OCH_3$ | $OCH_3$ | N | |
| 2.14 | Phenyl | 6-Cl | H | $C_2H_5$ | $OCH_3$ | $OCH_3$ | N | |
| 2.15 | Phenyl | 6-Cl | H | $C_2H_5$ | $CH_3$ | $OCH_3$ | N | |
| 2.16 | Phenyl | H | H | $CH(CH_3)_2$ | $CH_3$ | $OCH_3$ | CH | |
| 2.17 | Phenyl | H | H | $CH(CH_3)_2$ | $OCH_3$ | $OCH_3$ | CH | |
| 2.18 | Phenyl | H | H | $CH(CH_3)_2$ | $CH_3$ | $OCH_3$ | N | Smp.172-174°C |
| 2.19 | Phenyl | H | H | $CH(CH_3)_2$ | $OCH_3$ | $OCH_3$ | N | |
| 2.20 | Phenyl | H | H | $CH_2CH_2OCH_3$ | $CH_3$ | $OCH_3$ | CH | |
| 2.21 | Phenyl | H | H | $CH_2CH_2OCH_3$ | $OCH_3$ | $OCH_3$ | CH | |
| 2.22 | Phenyl | H | H | $CH_2CH_2OCH_3$ | $CH_3$ | $OCH_3$ | N | |
| 2.23 | Phenyl | H | H | $CH_2CH_2OCH_3$ | $OCH_3$ | $OCH_3$ | N | |
| 2.24 | Phenyl | 6-Cl | H | $CH_2CH_2OCH_3$ | $OCH_3$ | $OCH_3$ | N | |
| 2.25 | Phenyl | H | H | $CH_2COOCH_3$ | $CH_3$ | $OCH_3$ | CH | |
| 2.26 | Phenyl | H | H | $CH_2COOCH_3$ | $OCH_3$ | $OCH_3$ | CH | |
| 2.27 | Phenyl | H | H | $CH_2COOCH_3$ | $CH_3$ | $OCH_3$ | N | Smp.164-167°C |
| 2.28 | Phenyl | H | H | $CH_2COOCH_3$ | $OCH_3$ | $OCH_3$ | N | |
| 2.29 | Phenyl | H | $CH_3$ | $CH_2COOCH_3$ | $OCH_3$ | $OCH_3$ | N | |
| 2.30 | Phenyl | 6-Cl | H | $CH_2COOCH_3$ | $OCH_3$ | $OCH_3$ | N | |
| 2.31 | Phenyl | H | H | $CH(CH_3)COOCH_3$ | $CH_3$ | $OCH_3$ | CH | |
| 2.32 | Phenyl | H | H | $CH(CH_3)COOCH_3$ | $OCH_3$ | $OCH_3$ | CH | |

Tabelle 2 (Fortsetzung)

| Nr. | $R^2$ | $R^1$ | $R^3$ | $R^9$ | X | Y | E | Physikal. Daten |
|---|---|---|---|---|---|---|---|---|
| 2.33 | Phenyl | H | H | $CH(CH_3)COOCH_3$ | $CH_3$ | $OCH_3$ | N | |
| 2.34 | Phenyl | H | H | $CH(CH_3)COOCH_3$ | $OCH_3$ | $OCH_3$ | N | |
| 2.35 | Phenyl | H | H | $CH(CH_3)COOC_2H_5$ | $CH_3$ | $OCH_3$ | CH | |
| 2.36 | Phenyl | H | H | $CH(CH_3)COOC_2H_5$ | $OCH_3$ | $OCH_3$ | CH | |
| 2.37 | Phenyl | H | H | $CH(CH_3)COOC_2H_5$ | $CH_3$ | $OCH_3$ | N | |
| 2.38 | Phenyl | H | H | $CH(CH_3)COOC_2H_5$ | $OCH_3$ | $OCH_3$ | N | |
| 2.39 | Phenyl | 6-Cl | H | $CH(CH_3)COOC_2H_5$ | $OCH_3$ | $OCH_3$ | N | |
| 2.40 | Phenyl | H | H | $CH_2CH=CH_2$ | $CH_3$ | $OCH_3$ | CH | |
| 2.41 | Phenyl | H | H | $CH_2CH=CH_2$ | $OCH_3$ | $OCH_3$ | CH | |
| 2.42 | Phenyl | H | H | $CH_2CH=CH_2$ | $CH_3$ | $OCH_3$ | N | |
| 2.43 | Phenyl | H | H | $CH_2CH=CH_2$ | $OCH_3$ | $OCH_3$ | N | |
| 2.44 | Phenyl | 6-Cl | H | $CH_2CH=CH_2$ | $CH_3$ | $OCH_3$ | N | |
| 2.45 | Phenyl | H | H | $CH_2-CN$ | $CH_3$ | $OCH_3$ | CH | |
| 2.46 | Phenyl | H | H | $CH_2-CN$ | $OCH_3$ | $OCH_3$ | CH | |
| 2.47 | Phenyl | H | H | $CH_2-CN$ | $CH_3$ | $OCH_3$ | N | |
| 2.48 | Phenyl | H | H | $CH_2-CN$ | $OCH_3$ | $OCH_3$ | N | |
| 2.49 | Phenyl | 6-Cl | H | $CH_2-CN$ | $OCH_3$ | $OCH_3$ | N | |
| 2.50 | Phenyl | H | H | Benzyl | $CH_3$ | $OCH_3$ | CH | |
| 2.51 | Phenyl | H | H | Benzyl | $CH_3$ | $OCH_3$ | N | |
| 2.52 | Phenyl | H | H | 4-Chlorbenzyl | $CH_3$ | $OCH_3$ | N | |
| 2.53 | Phenyl | H | H | Phenyl | $CH_3$ | $OCH_3$ | CH | |
| 2.54 | Phenyl | H | H | Phenyl | $OCH_3$ | $OCH_3$ | N | Smp.156°C |
| 2.55 | Phenyl | H | H | $CH_2-CON(CH_3)_2$ | $CH_3$ | $OCH_3$ | CH | |

Tabelle 2 (Fortsetzung)

| Nr. | $R^2$ | $R^1$ | $R^3$ | $R^9$ | X | Y | E | Physikal. Daten |
|---|---|---|---|---|---|---|---|---|
| 2.56 | Phenyl | H | H | $CH_2$-$CON(CH_3)_2$ | $CH_3$ | $OCH_3$ | N | |
| 2.57 | Phenyl | H | H | $CH_2CF_3$ | $CH_3$ | $OCH_3$ | CH | |
| 2.58 | Phenyl | H | H | $CH_2CF_3$ | $OCH_3$ | $OCH_3$ | CH | |
| 2.59 | Phenyl | H | H | $CH_2CF_3$ | $CH_3$ | $OCH_3$ | N | |
| 2.60 | Phenyl | H | H | $CH_2CF_3$ | $OCH_3$ | $OCH_3$ | N | |
| 2.61 | 2-Fluor-phenyl | H | H | $CH_3$ | $CH_3$ | $OCH_3$ | CH | |
| 2.62 | 2-Fluor-phenyl | H | H | $CH_3$ | $CH_3$ | $OCH_3$ | N | |
| 2.63 | 2-Fluor-phenyl | H | H | $CH_3$ | $OCH_3$ | $OCH_3$ | N | |
| 2.64 | 2-Fluor-phenyl | H | H | $C_2H_5$ | $CH_3$ | $OCH_3$ | N | |
| 2.65 | 2-Fluor-phenyl | H | H | $C_2H_5$ | $OCH_3$ | $OCH_3$ | N | |
| 2.66 | 2-Fluor-phenyl | H | H | $CH_2CH_2OCH_3$ | $CH_3$ | $OCH_3$ | CH | |
| 2.67 | 2-Fluor-phenyl | H | H | $CH_2CH_2OCH_3$ | $OCH_3$ | $OCH_3$ | CH | |
| 2.68 | 2-Fluor-phenyl | H | H | $CH_2CH_2OCH_3$ | $CH_3$ | $OCH_3$ | N | |
| 2.69 | 2-Fluor-phenyl | H | H | $CH_2CH_2OCH_3$ | $OCH_3$ | $OCH_3$ | N | |
| 2.70 | Phenoxy | H | H | $CH_3$ | $CH_3$ | $OCH_3$ | N | |
| 2.71 | Phenoxy | H | H | $C_2H_5$ | $OCH_3$ | $OCH_3$ | N | |
| 2.72 | Phenoxy | H | H | $CH_2CH_2OCH_3$ | $OCH_3$ | $OCH_3$ | CH | |
| 2.73 | Benzyl | H | H | $CH_3$ | $OCH_3$ | $OCH_3$ | N | |
| 2.74 | Phenylthio | H | H | $CH_3$ | $OCH_3$ | $OCH_3$ | N | |

Tabelle 2 (Fortsetzung)

| Nr. | $R^2$ | $R^1$ | $R^3$ | $R^9$ | X | Y | E | Physikal. Daten |
|---|---|---|---|---|---|---|---|---|
| 2.75 | Phenyl | H | H | $CH_2COOH$ | $OCH_3$ | $OCH_3$ | CH | |
| 2.76 | Phenyl | H | H | $CH_2COOH$ | $OCH_3$ | $OCH_3$ | N | |
| 2.77 | Phenyl | H | H | $CH(CH_3)COOH$ | $OCH_3$ | $OCH_3$ | CH | |

Tabelle 3

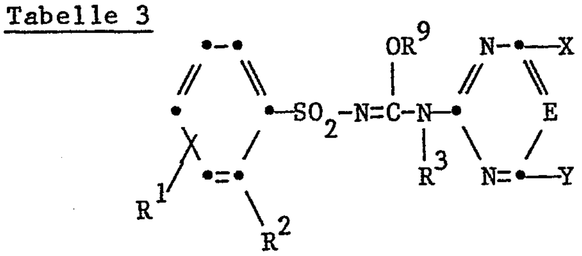

| Nr. | $R^2$ | $R^1$ | $R^3$ | $R^9$ | X | Y | E | Physikal. Daten |
|---|---|---|---|---|---|---|---|---|
| 3.01 | Phenyl | H | H | $CH_3$ | $CH_3$ | $OCH_3$ | CH | |
| 3.02 | Phenyl | H | H | $CH_3$ | $OCH_3$ | $OCH_3$ | CH | |
| 3.03 | Phenyl | H | H | $CH_3$ | $CH_3$ | $OCH_3$ | N | |
| 3.04 | Phenyl | H | H | $CH_3$ | $OCH_3$ | $OCH_3$ | N | |
| 3.05 | Phenyl | H | $CH_3$ | $CH_3$ | $OCH_3$ | $OCH_3$ | N | |
| 3.06 | Phenyl | 6-Cl | H | $CH_3$ | $OCH_3$ | $OCH_3$ | CH | |
| 3.07 | Phenyl | 6-Cl | H | $CH_3$ | $OCH_3$ | $OCH_3$ | N | |
| 3.08 | Phenyl | H | H | $C_2H_5$ | $CH_3$ | $OCH_3$ | CH | |
| 3.09 | Phenyl | H | H | $C_2H_5$ | $OCH_3$ | $OCH_3$ | CH | |
| 3.10 | Phenyl | H | H | $C_2H_5$ | $CH_3$ | $OCH_3$ | N | |
| 3.11 | Phenyl | H | H | $C_2H_5$ | $OCH_3$ | $OCH_3$ | N | |

Tabelle 3 (Fortsetzung)

| Nr. | $R^2$ | $R^1$ | $R^3$ | $R^9$ | X | Y | E | Physikal. Daten |
|---|---|---|---|---|---|---|---|---|
| 3.12 | Phenyl | 6-Cl | H | $C_2H_5$ | $OCH_3$ | $OCH_3$ | N | |
| 3.13 | Phenyl | H | H | $CH_2CH_2OCH_3$ | $CH_3$ | $OCH_3$ | CH | |
| 3.14 | Phenyl | H | H | $CH_2CH_2OCH_3$ | $OCH_3$ | $OCH_3$ | CH | |
| 3.15 | Phenyl | H | H | $CH_2CH_2OCH_3$ | $CH_3$ | $OCH_3$ | N | |
| 3.16 | Phenyl | H | H | $CH_2CH_2OCH_3$ | $OCH_3$ | $OCH_3$ | N | |
| 3.17 | Phenyl | H | $CH_3$ | $CH_2CH_2OCH_3$ | $OCH_3$ | $OCH_3$ | N | |
| 3.18 | Phenyl | 6-Cl | H | $CH_2CH_2OCH_3$ | $CH_3$ | $OCH_3$ | N | |
| 3.19 | Phenyl | 6-Cl | H | $CH_2CH_2OCH_3$ | $OCH_3$ | $OCH_3$ | N | |
| 3.20 | Phenyl | H | H | $CH_2COOCH_3$ | $CH_3$ | $OCH_3$ | CH | |
| 3.21 | Phenyl | H | H | $CH_2COOCH_3$ | $OCH_3$ | $OCH_3$ | CH | |
| 3.22 | Phenyl | H | H | $CH_2COOCH_3$ | $CH_3$ | $OCH_3$ | N | |
| 3.23 | Phenyl | H | H | $CH_2COOCH_3$ | $OCH_3$ | $OCH_3$ | N | |
| 3.24 | Phenyl | 6-Cl | H | $CH_2COOCH_3$ | $OCH_3$ | $OCH_3$ | N | |
| 3.25 | Phenyl | H | H | $CH(CH_3)COOC_3$ | $CH_3$ | $OCH_3$ | CH | |
| 3.26 | Phenyl | H | H | $CH(CH_3)COOCH_3$ | $OCH_3$ | $OCH_3$ | CH | |
| 3.27 | Phenyl | H | H | $CH(CH_3)COOCH_3$ | $CH_3$ | $OCH_3$ | N | |
| 3.28 | Phenyl | H | H | $CH(CH_3)COOCH_3$ | $OCH_3$ | $OCH_3$ | N | |
| 3.29 | Phenyl | 6-Cl | H | $CH(CH_3)COOCH_3$ | $OCH_3$ | $OCH_3$ | CH | |
| 3.30 | Phenyl | 6-Cl | H | $CH(CH_3)COOCH_3$ | $OCH_3$ | $OCH_3$ | N | |
| 3.31 | Phenyl | H | H | $CH(CH_3)COOC_2H_5$ | $OCH_3$ | $OCH_3$ | N | Smp.125°C (Zers.) |
| 3.32 | Phenyl | 6-Cl | H | $CH(CH_3)COOC_2H_5$ | $OCH_3$ | $OCH_3$ | N | |
| 3.33 | Phenyl | H | H | $CH_2CH=CH_2$ | $CH_3$ | $OCH_3$ | CH | |

Tabelle 3 (Fortsetzung)

| Nr. | $R^2$ | $R^1$ | $R^3$ | $R^9$ | X | Y | E | Physikal. Daten |
|---|---|---|---|---|---|---|---|---|
| 3.34 | Phenyl | H | H | $CH_2CH=CH_2$ | $OCH_3$ | $OCH_3$ | CH | |
| 3.35 | Phenyl | H | H | $CH_2CH=CH_2$ | $CH_3$ | $OCH_3$ | N | Smp.112° |
| 3.36 | Phenyl | H | H | $CH_2CH=CH_2$ | $OCH_3$ | $OCH_3$ | N | |
| 3.37 | Phenyl | 6-Cl | H | $CH_2CH=CH_2$ | $OCH_3$ | $OCH_3$ | N | |
| 3.38 | Phenyl | H | H | $CH_2$-CN | $CH_3$ | $OCH_3$ | CH | |
| 3.39 | Phenyl | H | H | $CH_2$-CN | $OCH_3$ | $OCH_3$ | CH | |
| 3.40 | Phenyl | H | H | $CH_2$-CN | $CH_3$ | $OCH_3$ | N | |
| 3.41 | Phenyl | H | H | $CH_2$-CN | $OCH_3$ | $OCH_3$ | N | |
| 3.42 | Phenyl | 6-Cl | H | $CH_2$-CN | $OCH_3$ | $OCH_3$ | CH | |
| 3.43 | Phenyl | 6-Cl | H | $CH_2$-CN | $OCH_3$ | $OCH_3$ | N | |
| 3.44 | Phenyl | H | H | Benzyl | $CH_3$ | $OCH_3$ | N | Smp. 137°C |
| 3.45 | Phenyl | H | H | Benzyl | $OCH_3$ | $OCH_3$ | CH | |
| 3.46 | Phenyl | H | H | Benzyl | $OCH_3$ | $OCH_3$ | N | |
| 3.47 | Phenyl | H | H | 4-Chlorbenzyl | $OCH_3$ | $OCH_3$ | N | |
| 3.48 | Phenyl | H | H | Phenyl | $OCH_3$ | $OCH_3$ | N | Smp. 143°C (Zers.) |
| 3.49 | Phenyl | H | H | $CH_2-CON(CH_3)_2$ | $OCH_3$ | $OCH_3$ | N | |
| 3.50 | Phenyl | H | H | $CH_2CF_3$ | $CH_3$ | $OCH_3$ | CH | |
| 3.51 | Phenyl | H | H | $CH_2CF_3$ | $OCH_3$ | $OCH_3$ | CH | |
| 3.52 | Phenyl | H | H | $CH_2CF_3$ | $CH_3$ | $OCH_3$ | N | |
| 3.53 | Phenyl | H | H | $CH_2CF_3$ | $OCH_3$ | $OCH_3$ | N | |
| 3.54 | Phenyl | 6-Cl | H | $CH_2CF_3$ | $OCH_3$ | $OCH_3$ | N | |
| 3.55 | 2-Fluor-phenyl | H | H | $CH_3$ | $CH_3$ | $OCH_3$ | CH | |

Tabelle 3 (Fortsetzung)

| Nr. | $R^2$ | $R^1$ | $R^3$ | $R^9$ | X | Y | E | Physikal. Daten |
|---|---|---|---|---|---|---|---|---|
| 3.56 | 2-Fluor-phenyl | H | H | $CH_3$ | $OCH_3$ | $OCH_3$ | CH | |
| 3.57 | 2-Fluor-phenyl | H | H | $CH_3$ | $CH_3$ | $OCH_3$ | N | |
| 3.58 | 2-Fluor-phenyl | H | H | $CH_3$ | $OCH_3$ | $OCH_3$ | N | |
| 3.59 | 2-Fluor-phenyl | 6-Cl | H | $CH_3$ | $OCH_3$ | $OCH_3$ | N | |
| 3.60 | 2-Fluor-phenyl | 6-Cl | H | $CH_3$ | $CH_3$ | $OCH_3$ | N | |
| 3.61 | 2-Fluor-phenyl | H | H | $CH_2CH_2OCH_3$ | $OCH_3$ | $OCH_3$ | N | |
| 3.62 | 2-Fluor-phenyl | H | H | $CH_2COOC_2H_5$ | $OCH_3$ | $OCH_3$ | N | |
| 3.63 | 2-Fluor-phenyl | H | H | $CH(CH_3)COOCH_3$ | $OCH_3$ | $OCH_3$ | N | |
| 3.64 | 2-Fluor-phenyl | H | H | $CH_2CH{=}CH_2$ | $OCH_3$ | $OCH_3$ | CH | |
| 3.65 | Phenoxy | H | H | $CH_3$ | $CH_3$ | $OCH_3$ | CH | |
| 3.66 | Phenoxy | H | H | $CH_3$ | $OCH_3$ | $OCH_3$ | CH | |
| 3.67 | Phenoxy | H | H | $CH_3$ | $CH_3$ | $OCH_3$ | N | |
| 3.68 | Phenoxy | H | H | $CH_3$ | $OCH_3$ | $OCH_3$ | N | |
| 3.69 | Phenoxy | 6-Cl | H | $CH_3$ | $OCH_3$ | $OCH_3$ | N | |
| 3.70 | Phenoxy | H | H | $CH_2CH_2OCH_3$ | $OCH_3$ | $OCH_3$ | N | |
| 3.71 | Phenoxy | H | H | $CH_2COOC_2H_5$ | $OCH_3$ | $OCH_3$ | N | |
| 3.72 | Phenoxy | H | H | $CH(CH_3)COOCH_3$ | $OCH_3$ | $OCH_3$ | CH | |
| 3.73 | Phenoxy | H | H | $CH_2CH{=}CH_2$ | $OCH_3$ | $OCH_3$ | N | |

Tabelle 3 (Fortsetzung)

| Nr. | $R^2$ | $R^1$ | $R^3$ | $R^9$ | X | Y | E | Physikal. Daten |
|---|---|---|---|---|---|---|---|---|
| 3.74 | Phenoxy | H | H | $CH_2-CN$ | $OCH_3$ | $OCH_3$ | CH | |
| 3.75 | Phenoxy | H | H | $CH_2-CN$ | $OCH_3$ | $OCH_3$ | N | |
| 3.76 | Phenoxy | 6-Cl | H | $CH_2-CN$ | $OCH_3$ | $OCH_3$ | N | |
| 3.77 | Phenoxy | H | $CH_3$ | $CH_2-CN$ | $OCH_3$ | $OCH_3$ | N | |
| 3.78 | Benzyl | H | H | $CH_3$ | $CH_3$ | $OCH_3$ | CH | |
| 3.79 | Benzyl | H | H | $CH_3$ | $OCH_3$ | $OCH_3$ | N | |
| 3.80 | Benzyl | H | H | $CH_3$ | $CH_3$ | $OCH_3$ | N | |
| 3.81 | Benzyl | H | H | $CH_2CH_2OCH_3$ | $OCH_3$ | $OCH_3$ | CH | |
| 3.82 | Benzyl | H | H | $CH_2CH_2OCH_3$ | $OCH_3$ | $OCH_3$ | N | |
| 3.83 | Benzyl | H | H | $CH_2COOC_2H_5$ | $OCH_3$ | $OCH_3$ | N | |
| 3.84 | Benzyl | H | H | $CH(CH_3)COOCH_3$ | $OCH_3$ | $OCH_3$ | CH | |
| 3.85 | Benzyl | H | H | $CH(CH_3)COOCH_3$ | $OCH_3$ | $OCH_3$ | N | |
| 3.86 | Benzyl | H | H | $CH_2CH=CH_2$ | $OCH_3$ | $OCH_3$ | N | |
| 3.87 | Benzyl | H | H | $CH_2-CN$ | $OCH_3$ | $OCH_3$ | N | |
| 3.88 | Benzyl | 6-Cl | H | $CH_3$ | $OCH_3$ | $OCH_3$ | CH | |
| 3.89 | Benzyl | 6-Cl | H | $CH_3$ | $OCH_3$ | $OCH_3$ | N | |
| 3.90 | Phenyl | H | H | $CH_3$ | $OCHF_2$ | $OCH_3$ | CH | |
| 3.91 | Phenyl | 6-Cl | H | $CH_3$ | $OCHF_2$ | $OCH_3$ | CH | |
| 3.92 | Phenyl | 6-Cl | H | $CH_3$ | $OCHF_2$ | $CH_3$ | CH | |
| 3.93 | Phenyl | H | H | $-(CH_2)_2N(CH_3)_2$ | $OCH_3$ | $OCH_3$ | CH | |
| 3.94 | Phenyl | H | H | $CH(CH_3)_2$ | $CH_3$ | $OCH_3$ | N | Smp.140-142°C (Zers.) |

Tabelle 4

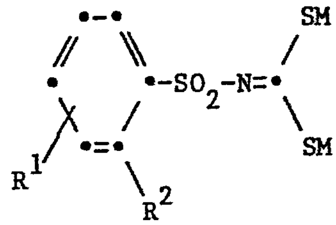

| Nr. | $R^2$ | R' | M | Physikal. Daten |
|---|---|---|---|---|
| 4.01 | Phenyl | H | K | Smp. 245°C (Zers.) |
| 4.02 | 2-Fluorphenyl | H | K | |
| 4.03 | Phenoxy | H | K | |
| 4.04 | 2-Fluorphenoxy | H | K | |
| 4.05 | Benzyl | H | K | |
| 4.06 | Phenyl | 6-Cl | K | |
| 4.07 | Phenyl | H | Na | |
| 4.08 | Phenyl | $6-OCH_3$ | K | |

Tabelle 5

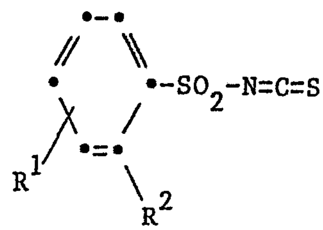

| Nr. | $R^2$ | $R^1$ |
|---|---|---|
| 5.01 | Phenyl | H |
| 5.02 | 2-Fluorphenyl | H |
| 5.03 | Phenoxy | H |
| 5.04 | 2-Fluorphenoxy | H |
| 5.05 | Benzyl | H |
| 5.06 | Phenyl | 6-Cl |
| 5.07 | Phenyl | 6-$OCH_3$ |

Tabelle 6

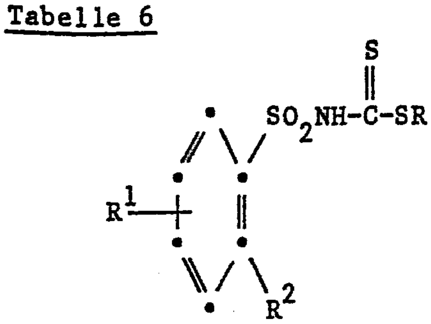

| Nr. | $R^2$ | $R^1$ | R |
|---|---|---|---|
| 6.01 | Phenyl | H | $CH_3$ |
| 6.02 | Phenyl | H | $C_2C_5$ |
| 6.03 | Phenyl | H | $n-C_4H_9$ |
| 6.04 | Phenyl | H | $CH_2C_6H_5$ |
| 6.05 | Phenyl | H | $CH_2CH=CH_2$ |
| 6.06 | Phenyl | H | $CH_2CO_2C_2H_5$ |
| 6.07 | Phenyl | H | $CH_2COCH_3$ |
| 6.08 | Phenyl | 6-Cl | $CH_3$ |
| 6.09 | 2-Fluorphenyl | H | $CH_3$ |
| 6.10 | 3-Fluorphenyl | H | $CH_3$ |
| 6.11 | 4-Fluorphenyl | H | $CH_3$ |
| 6.12 | Phenoxy | H | $CH_3$ |
| 6.13 | 2-Fluorphenoxy | H | $CH_3$ |
| 6.14 | 3-Fluorphenoxy | H | $CH_3$ |
| 6.15 | 4-Fluorphenoxy | H | $CH_3$ |
| 6.16 | Benzyl | H | $CH_3$ |
| 6.17 | 2-Chlorbenzyl | H | $CH_3$ |
| 6.18 | 3-Chlorbenzyl | H | $CH_3$ |
| 6.19 | 4-Chlorbenzyl | H | $CH_3$ |

Tabelle 7

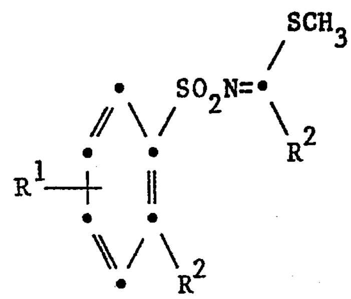

| Nr. | $R^2$ | $R^1$ | $R^9$ |
|---|---|---|---|
| 7.01 | Phenyl | H | $CH_3$ |
| 7.02 | Phenyl | H | $C_2H^5$ |
| 7.03 | Phenyl | H | $CH_2CO_2C_2H_5$ |
| 7.04 | Phenyl | H | $CH_2C_6H_5$ |
| 7.05 | 2-Fluorphenyl | H | $CH_3$ |
| 7.06 | Phenoxy | H | $CH_3$ |
| 7.07 | Phenyl | 6-Cl | $CH_3$ |
| 7.08 | Benzyl | H | $CH_3$ |

Formulierungsbeispiele:

Beispiel F 1: Formulierungsbeispiele für Wirkstoffe der Formel I (% = Gewichtsprozent)

| a) Spritzpulver | a) | b) | c) |
|---|---|---|---|
| Wirkstoff | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther /7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Aethylenglykol | 10 % | 10 % |
| Nonylphenolpolyäthylenglykoläther (15 MOl AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

Biologische Beispiele:

Beispiel B 1: Herbizidwirkung vor dem Auflaufen der Pflanzen

Kunststofftöpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis und Stellaria media. Die Versuchsgefässe werden anschliessend in einer Klimakammer bei 20°C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze / Wirkstoff Nr | Nasturtium officinalis | Stellaria media | Agrostis tenuis |
|---|---|---|---|
| 1.02 | 1 | 2 | 1 |
| 1.03 | 1 | 2 | 1 |
| 2.03 | 2 | 2 | 7 |
| 3.94 | 2 | 4 | 3 |
| 2.12 | 4 | 4 | 4 |
| 3.35 | 2 | 3 | 3 |
| 3.44 | 2 | 4 | 2 |
| 3.48 | 2 | 3 | 4 |
| 3.31 | 3 | 3 | 3 |

Beispiel B 2: Wuchshemmung bei tropischen Bodendecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel B 3: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klima kammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B 4: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B 5: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

Patentansprüche

1. Phenylsulfonamidderivate der Formel I

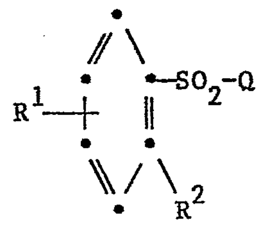

(I)

worin

$R^1$ Wasserstoff, Fluor, Chlor, Brom, Nitro, $C_1$-$C_4$-Alkyl, $C_1$-$C_4$-Alkylthio, $C_1$-$C_4$-Alkoxy, Trifluormethyl, Trifluormethoxy, Difluormethoxy oder $C_1$-$C_4$-Alkoxycarbonyl,

$R^2$ eine Gruppe 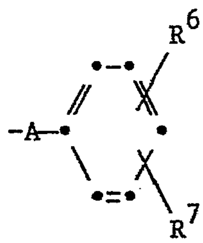 ,

Q eine Gruppe 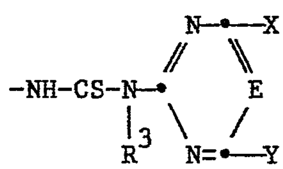 oder

eine Gruppe 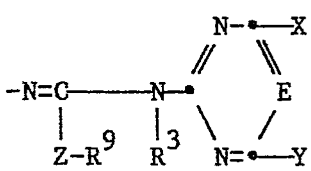 ,

E Stickstoff oder die Methingruppe,

Z Sauerstoff oder Schwefel,

A Sauerstoff, Schwefel, eine -SO- oder $-SO_2$-Brücke oder eine Gruppe

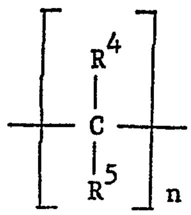

,

n die Zahl eins oder null,

$R^3$ Wasserstoff oder $C_1$-$C_4$-Alkyl,

$R^4$ und $R^5$ unabhängig voneinander Wasserstoff oder $C_1$-$C_4$-Alkyl,

$R^6$ Wasserstoff, $C_1$-$C_4$-Alkyl, $C_1$-$C_4$-Alkoxy, Halogen, Trifluormethyl, Nitro, $COOR^8$ oder durch Halogen substituiertes $C_1$-$C_4$-Alkoxy,

$R^7$ Wasserstoff, Fluor, Chlor, Brom, $C_1$-$C_4$-Alkyl oder $C_1$-$C_4$-Alkoxy,

$R^9$ $C_1$-$C_6$-Alkyl, $C_3$-$C_6$-Alkenyl, $C_3$-$C_6$-Alkinyl, $C_2$-$C_6$-Halogenalkyl, $C_2$-$C_6$-Alkoxyalkyl, Benzyl, Phenyl, eine Gruppe $-CHR^{10}-COOR^{11}$, $-CHR^{10}-CN$, $-CHR^{10}-CONR^{12}R^{13}$, $-(CH_2)_m-NR^{12}R^{13}$ oder durch $C_1$-$C_4$-Alkyl, Fluor, Chlor, Brom, Nitro, Trifluormethyl, Trifluormethoxy oder $C_1$-$C_4$-Alkoxy substituiertes Benzyl oder Phenyl,

m eine Zahl von zwei bis sechs,

$R^{10}$ Wasserstoff oder $C_1$-$C_4$-Alkyl,

$R^8$ und $R^{11}$ unabhängig voneinander Wasserstoff, $C_1$-$C_4$-Alkyl, $C_2$-$C_4$-Alkenyl, $C_3$-$C_4$-Alkinyl oder durch $C_1$-$C_4$-Alkoxy, Fluor, Chlor, Brom oder Phenyl substituiertes $C_1$-$C_4$-Alkyl,

$R^{12}$ Wasserstoff, $C_1$-$C_4$-Alkyl, $C_2$-$C_4$-Alkenyl oder $C_3$-$C_4$-Alkinyl,

$R^{13}$ Wasserstoff, $C_1$-$C_4$-Alkyl, $C_2$-$C_4$-Alkenyl, $C_3$-$C_4$-Alkinyl, $C_1$-$C_4$-Alkoxy, $C_3$-$C_6$-Cycloalkyl, Phenyl oder Benzyl,

$R^{14}$ und $R^{15}$ unabhängig voneinander Wasserstoff oder $C_1$-$C_4$-Alkyl und

X und Y unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder $-NR^{14}R^{15}$ bedeuten,

mit der Massgabe, dass keiner der Reste X und Y für Difluormethoxy steht, wenn die Gruppe Q für

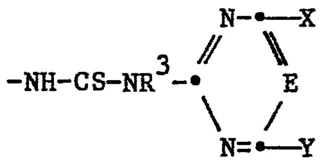 steht,

sowie die Salze dieser Verbindungen.

2. Verbindungen gemäss Anspruch 1, dadurch gekennzeichnet, dass $R^1$ für Wasserstoff oder Chlor steht.

3. Verbindungen gemäss Anspruch 1, dadurch gekennzeichnet, dass $R^3$ für Wasserstoff oder Methyl steht.

4. Verbindungen gemäss Anspruch 1, dadurch gekennzeichnet, dass $R^6$ für Wasserstoff oder Halogen und $R^7$ für Wasserstoff steht.

5. Verbindungen gemäss Anspruch 1, dadurch gekennzeichnet, dass X und Y unabhängig voneinander $C_1$-$C_4$-Alkyl, $C_1$-$C_4$-Alkoxy oder $C_1$-$C_4$-Halogenalkoxy bedeuten und zusammen höchstens 5 Kohlenstoffatome enthalten.

6. Verbindungen gemäss Anspruch 5, dadurch gekennzeichnet, dass X und Y unabhängig voneinander Methyl oder Methoxy bedeuten.

7. Verbindungen gemäss Anspruch 1, dadurch gekennzeichnet, dass A die direkte Bindung, Sauerstoff oder eine Methylenbrücke bedeutet.

8. Verbindungen gemäss Anspruch 1, dadurch gekennzeichnet, dass $R^1$ Wasserstoff oder Chlor, $R^3$ Wasserstoff oder Methyl, $R^6$ Wasserstoff oder Halogen, $R^7$ Wasserstoff, A die direkte Bindung, Sauerstoff oder eine Methylenbrücke und X und Y unabhängig voneinander $C_1$-$C_4$-Alkyl, $C_1$-$C_4$-Alkoxy oder $C_1$-$C_4$-Halogenalkoxy, wobei X und Y zusammen höchstens 5 Kohlenstoffatome enthalten, bedeuten.

9. N-(2-Phenyl-phenylsulfonyl)-N'-(4,6-dimethoxypyrimidin-2-yl)-S-methyl-isothioharnstoff gemäss Anspruch 1.

10. N-(2-Phenyl-phenylsulfonyl)-N'-(4,6-dimethoxypyrimidin-2-yl)-thioharnstoff gemäss Anspruch 1.

11. N-(2-Phenyl-phenylsulfonyl)-N'-(4-methoxy-6-methyl-pyrimidin-2-yl)-thioharnstoff gemäss Anspruch 1.

12. N-(2-Phenyl-phenylsulfonyl)-N'-(4,6-dimethoxy-triazin-2-yl)-thioharnstoff gemäss Anspruch 1.

13. N-(2-Phenyl-phenylsulfonyl)-N'-(4-methoxy-6-methyl-triazin-2-yl)-thioharnstoff gemäss Anspruch 1.

14. Sulfonylimino-dithio-carbonate der Formel XIV

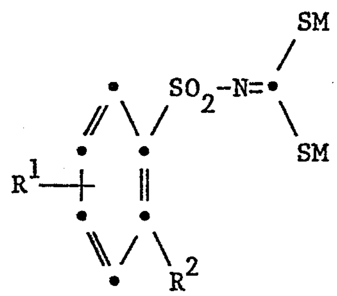

(XIV)

worin $R^1$ und $R^2$ die unter Formel I im Anspruch 1 gegebenen Bedeutungen haben und M für ein Metallatom oder ein Aequivalent eines Metallatoms, vorzugsweise Lithium, Natrium, Kalium, Calcium oder Magnesium, steht.

15. Sulfonylisothiocyanate der Formel VI

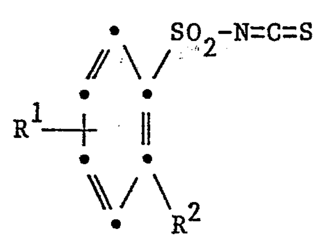

(VI)

worin $R^1$ und $R^2$ die unter Formel I im Anspruch 1 gegebenen Bedeutungen haben.

16. Carbodiimide der Formel VIII

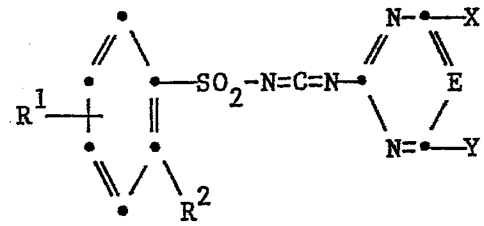

(VIII)

worin $R^1$, $R^2$, E, X und Y die unter Formel I im Anspruch 1 gegebenen Bedeutungen haben.

17. Verfahren zur Herstellung der Verbindungen der Formel Ia

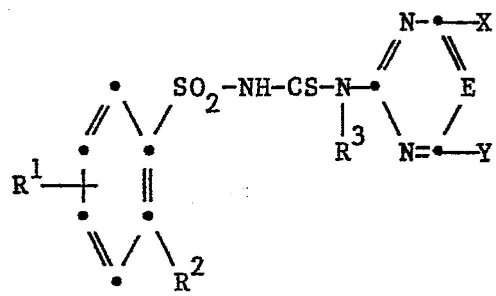

(Ia),

worin $R^1$, $R^2$, $R^3$, E, X und Y die unter Formel I gegebenen Bedeutungen haben, dadurch gekennzeichnet, dass man

a) ein substituiertes Phenylsulfonamid der Formel II

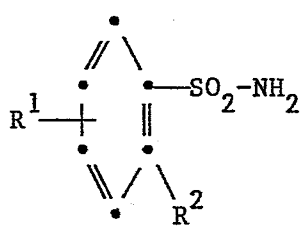

(II)

worin $R^1$ und $R^2$ die unter Formel I gegebenen Bedeutungen haben, in Gegenwart einer Base mit einem Dithiocarbamat der Formel III

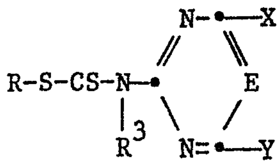 (III),

worin $R^3$, E, X und Y die unter Formel I gegebenen Bedeutungen haben und R für Phenyl, Alkyl oder substituiertes Phenyl steht, umsetzt oder

b) ein Phenylsulfondithiocarbamat der Formel IV

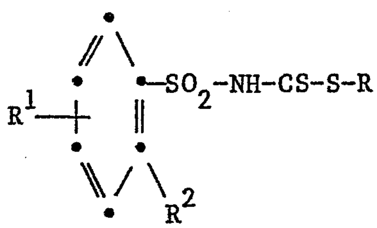 (IV),

worin $R^1$ und $R^2$ die unter Formel I gegebenen Bedeutungen haben und R für Phenyl, Alkyl oder substituiertes Phenyl steht, mit einem Amin der Formel V

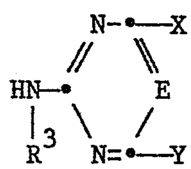 (V),

worin $R^3$, E, X und Y die unter Formel I gegebenen Bedeutungen haben, umsetzt, oder

c) ein Sulfonylisothiocyanat der Formel VI

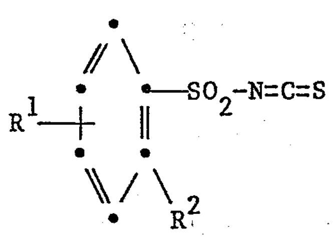 (VI),

0151554

worin $R^1$ und $R^2$ die unter Formel I gegebenen Bedeutungen haben, mit einem Amin der oben angegebenen Formel V umsetzt.

18. Verfahren zur Herstellung der Verbindungen der Formel Ib

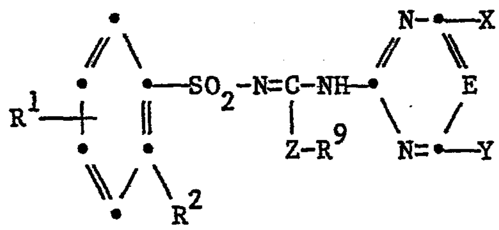

(Ib) ,

worin $R^1$, $R^2$, $R^9$, E, X, Y und Z die unter Formel I gegebenen Bedeutungen haben, dadurch gekennzeichnet, dass man einen Sulfonylharnstoff der Formel VII

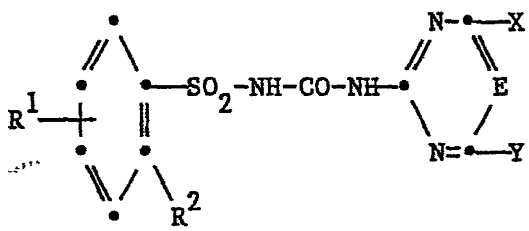

(VII),

worin $R^1$, $R^2$ und E, X und Y die unter Formel I gegebenen Bedeutungen haben, durch Umsetzung mit einem Dehydratisierungsreagenz in ein Carbodiimid der Formel VIII

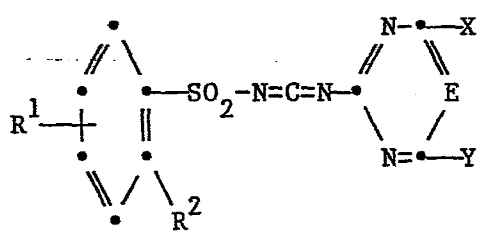

(VIII),

worin $R^1$, $R^2$, E, X und Y die unter Formel I gegebenen Bedeutungen haben, überführt und dieses mit einem Alkohol oder Mercaptan der Formel IX

$$H - Z - R^9 \qquad (IX)$$

worin $R^9$ und Z die unter Formel I gegebenen Bedeutungen haben, umsetzt.

19. Verfahren zur Herstellung von Additionssalzen der Formel I gemäss einem der Ansprüche 17 oder 18, dadurch gekennzeichnet, dass man ein Phenylsulfonamidderivat der Formel I mit einem Amin, einem Alkalimetall- oder Erdalkalimetallhydroxid oder einer quaternären Ammoniumbase umsetzt.

20. Ein herbizides und den Pflanzenwuchs hemmendes Mittel, dadurch gekennzeichnet, dass es neben Träger- und/oder anderen Zuschlagstoffen als Wirkstoff mindestens ein Phenylsulfonamidderivat der Formel I, Anspruch 1, enthält.

21. Die Verwendung der Wirkstoffe der Formel I, gemäss Anspruch 1, oder sie enthaltender Mittel zur Bekämpfung unerwünschten Pflanzenwachstums.

22. Die Verwendung der Wirkstoffe der Formel I, gemäss Anspruch 1, oder sie enthaltender Mittel zur Hemmung des Pflanzenwachstums.

23. Die Verwendung der Wirkstoff der Formel I, gemäss Anspruch 1, oder sie enthaltender Mittel zur Beeinflussung des Pflanzenwachstums zum Zweck der Ertragssteigerung.

24. Die Verwendung der Wirkstoffe der Formel I, oder sie enthaltender Mittel, gemäss Anspruch 21, zur selektiven pre- oder postemergenten Bekämpfung von Unkräutern in Nutzpflanzenkulturen.

25. Die Verwendung der Wirkstoffe gemäss Anspruch 22, zur Unterdrückung des Pflanzenwachstums über das 2-Blattstadium hinaus, dadurch gekennzeichnet, dass die Wirkstoffe preemergent angewendet werden.

26. Die Verwendung gemäss Anspruch 24 in Kulturen von Reis.

FO 7.5/CW/am*/cw*

0151554

Europäisches Patentamt

# EUROPÄISCHER RECHERCHENBERICHT

Nummer der Anmeldung

EP 85 81 0028

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,Y | EP-A-0 044 211 (E.I. DU PONT DE NEMOURS & CO.)<br>* Ansprüche * | 1,21 | C 07 D 239/46<br>C 07 D 239/52<br>C 07 D 251/16<br>C 07 D 251/46<br>A 01 N 47/36 //<br>C 07 C 161/00<br>C 07 C 161/04 |
| D,Y | EP-A-0 056 969 (NIHON TOKUSHU NOYAKU SEIZO K.K.)<br>* Ansprüche * | 1,21 | |
| D,Y | EP-A-0 074 595 (NIHON TOKUSHU NOYAKU SEIZO K.K.)<br>* Ansprüche * | 1,21 | |
| Y | EP-A-0 094 790 (E.I. DU PONT DE NEMOURS & CO.)<br>* Ansprüche * | 1,21 | |
| D,Y | EP-A-0 044 807 (CIBA-GEIGY AG)<br>* Ansprüche * | 1,21 | RECHERCHIERTE SACHGEBIETE (Int. Cl.4) |
| Y | EP-A-0 024 215 (E.I. DU PONT DE NEMOURS & CO.)<br>* Ansprüche * | 1,21 | C 07 D 239/00<br>C 07 D 251/00<br>C 07 C 161/00 |
| Y | EP-A-0 005 986 (E.I. DU PONT DE NEMOURS & CO.)<br>* Ansprüche * | 1,21 | |
| Y | EP-A-0 072 347 (CIBA-GEIGY)<br>* Seite 42, Verbindung 415 * | 1,21 | |

Der vorliegende Recherchenbericht wurde für alle Patentansprüche erstellt.

| Recherchenort | Abschlußdatum der Recherche | Prüfer |
|---|---|---|
| DEN HAAG | 22-04-1985 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82